# EUROPÄISCHE PATENTANMELDUNG

(11) **EP 1 557 127 A1**
(43) Veröffentlichungstag der Anmeldung: **27.07.2005**
(21) Anmeldenummer: 05405023.2
(22) Anmeldetag: 14.01.2005
(51) Int. Cl.: A61B 17/11, A61B 17/115

(54) **Chirurgisches Instrument zum Umstülpen eines Hohlorgans**

(30) Priorität: 22.01.2004 CH 812004
(71) Anmelder: Zürcher Hochschule Winterthur, 8401 Winterthur (CH)
(72) Erfinder: Nef, Thomas, 8173 Neerach (CH); Medricky, Petr, 8404 Winterthur (CH); Von Mentlen, Roger, 8427 Rorbas (CH); Brom, Charles, 8400 Winterthur (CH)
(74) Vertreter: Patentanwälte Breiter + Wiedmer AG

(57) **Zusammenfassung**

Ein chirurgisches Instrument zum Umstülpen eines aus einem von einander gegenüberstehenden und einen Durchführungsschlitz begrenzenden Stegen gebildeten Amboss herausragenden endständigen Stumpfes eines Hohlorgans über die freien Enden der Stege nach aussen weist zwei von freien Enden (16,18) von zwei eine Längsrichtung (x) definierenden Schenkeln (12,14) abragende und in einer gemeinsamen Ebene liegende Stifte (20,22) auf. Ein erster Schenkel (12) ist über einen in einer im zweiten Schenkel (14) in der Längsrichtung (x) angeordneten Führungsnut (28) gelagerten Führungsbolzen (30) mit senkrecht zur Stiftebene liegender Bolzenachse mit dem zweiten Schenkel (14) so verbunden, dass der zweite Schenkel (14) um die Bolzenachse drehbar und relativ zum ersten Schenkel (12) in Längsrichtung (x) verschiebbar ist.

## Beschreibung

Die Erfindung betrifft ein chirurgisches Instrument zum Umstülpen eines aus einem von einander gegenüberstehenden und einen Durchführungsschlitz begrenzenden Stegen gebildeten Amboss herausragenden endständigen Stumpfes eines Hohlorgans über die freien Enden der Stege nach aussen.

Bei der Durchführung einer Anastomose mit einem aus der unveröffentlichten schweizerischen Patentanmeldung bekannten Apparat wird der endständige Stumpf eines ersten Hohlorgans am Austrittsende eines von einander gegenüberstehenden Stegen begrenzten Durchführungsschlitzes über die freien Enden der einen Amboss bildenden Stege nach aussen umgestülpt. Anschliessend wird eine Seitenwand eines zweiten Hohlorgans mit einer Öffnung über den umgestülpten endständigen Stumpf des ersten Hohlorgans gelegt und die beiden Hohlorgane werden im Überlappungsbereich miteinander vernäht.

Aus der CH-A-557 672 ist eine chirurgische Vorrichtung mit Mitteln zum Umstülpen von Hohlorganen mittels eines von einer zweiteiligen Hülse gebildeten Ambosses bekannt. Nach dem Einlegen des umzustülpenden Hohlorgans in die Hülse wird der Umstülpvorgang mit Hilfe einer Pinzette durchgeführt.

Der Erfindung liegt die Aufgabe zugrunde, ein chirurgisches Instrument der eingangs genannten Art zu schaffen, mit dem das Umstülpen auf einfache Weise und mit wenigen Handgriffen durchgeführt werden kann.

Zur erfindungsgemässen Lösung der Aufgabe führt ein chirurgisches Instrument mit zwei von freien Enden von zwei eine Längsrichtung definierenden Schenkeln abragenden und in einer gemeinsamen Ebene liegenden Stiften, wobei ein erster Schenkel über einen in einer im zweiten Schenkel in Längsrichtung angeordneten Führungsnut gelagerten Führungsbolzen mit senkrecht zur Stiftebene liegender Bolzenachse mit dem zweiten Schenkel so verbunden ist, dass der zweite Schenkel um die Bolzenachse drehbar und relativ zum ersten Schenkel in Längsrichtung verschiebbar ist.

Mit dem erfindungsgemässen chirurgischen Instrument lassen sich alle zur Durchführung des Umstülpvorganges erforderliche Bewegungen ausführen.

Bevorzugt sind die beiden Schenkel im Bereich zwischen dem Führungsbolzen und den freien Enden der Schenkel über ein elastisches oder federndes Rückstellelement mit einer die beiden Schenkel auf einander zu drückenden Rückstellkraft beaufschlagt. Im einfachsten Fall ist das Rückstellelement ein die beiden Schenkel umschliessender Gummiring.

Zweckmässigerweise sind die beiden Schenkel über ein Verbindungselement miteinander verbunden, wobei das Verbindungselement mit dem ersten Schenkel starr und mit dem zweiten Schenkel über den am Verbindungselement festgelegten und in die Führungsnut eingreifenden Führungsbolzen verbunden ist.

Bevorzugt sind die Stifte an den freien Enden der Schenkel auswechselbar angeordnet.

In einer Nullstellung berühren sich die Spitzen der Stifte vorzugsweise unter Bildung einer V-Form.

Um ein Abgleiten eines von den Stiften gehaltenen Hohlorgans zu vermeiden, weisen die Stifte im Bereich ihrer Spitze auf der von einander weg weisenden Seite eine Ausnehmung mit einer im Wesentlichen ebenen Auflagefläche auf.

Für eine optimale Handhabung kann der erste Schenkel an seinem dem Stift fernen Ende einen Ring zur Aufnahme eines Zeig- oder Mittelfingers aufweisen. In diesem Fall ist der zweite Schenkel an seinem dem Stift fernen Ende zweckmässigerweise mit einem durch einen Daumen bedienbaren Griffteil zur Ausführung von Translationsbewegungen des zweiten Schenkels in Längsrichtung ausgestattet.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt schematisch in
- - Fig. 1: eine Schrägsicht auf ein chirurgisches Instrument in Nullstellung;
- - Fig. 2: eine Draufsicht auf das chirurgische Instrument von Fig. 1 in Nullstellung;
- - Fig. 3: das chirurgische Instrument von Fig. 2 mit gespreizten Stiften;
- - Fig. 4: das chirurgische Instrument von Fig. 3 mit einem Stift in vorgeschobener Position;
- - Fig. 5: eine Schrägsicht auf einen Amboss zur Durchführung und Umstülpung eines Hohlorgans;
- - Fig. 6 - 9: die mit dem chirurgischen Instrument von Fig. 1 durchzuführenden Schritte beim Umstülpen eines Hohlorgans über einen Amboss.

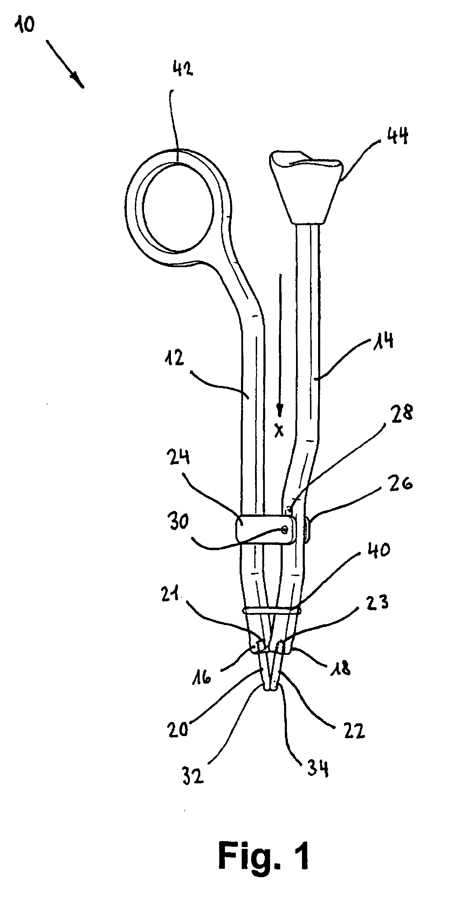

Ein in Fig. 1 dargestelltes chirurgisches Instrument 10 einer beispielsweisen Länge von etwa 15 cm weist zwei Schenkel 12, 14 mit von deren freien Enden 16, 18 abragenden Stiften 20, 22 mit einem Durchmesser von z.B. 2 mm auf. Die Stifte 20,22 weisen im Bereich ihrer Spitze auf der von einander weg weisenden Seite eine Ausnehmung 32,34 mit einer im Wesentlichen ebenen Auflagefläche auf. Diese Massnahme verhindert beim Umstülpen eines Hohlorgans ein vorzeitiges Abgleiten des Randes vom Stift. Die Stifte 20,22 sind über ein Schraubengewinde 21,23 mit den Schenkeln 12,14 verschraubt und auf diese Weise austauschbar.

Zwei Verbindungsplatten 24, 26 liegen gegenüberliegenden Seiten eines ersten Schenkels 12 an und sind mit diesem starr verbunden. Im zweiten Schenkel 14 ist eine Führungsnut 28 einer Länge von beispielsweise 5 mm in der durch die beiden Schenkel 12, 14 definierten allgemeinen Längsrichtung x angeordnet.

Der zweite Schenkel 14 wird im Bereich der Führungsnut 28 von den beiden vom ersten Schenkel 12 abragenden Verbindungsplatten 24, 26 übergriffen. Ein Führungsbolzen 30 verbindet die beiden Verbindungsplatten 24, 26 und durchsetzt die Führungsnut 28. Mit dieser Anordnung lässt sich der zweite Schenkel 14 um die Bolzenachse a schwenken, wodurch die beiden Stifte 20, 22 auseinandergespreizt werden. Die Führungsnut 28 ermöglicht eine Translationsbewegung des zweiten Schenkels 14 in Längsrichtung x relativ zum ersten Schenkel 12.

Im Bereich zwischen den Verbindungsplatten 24, 26 und den freien Enden 16, 18 werden die Schenkel 12, 14 von einem elastisch gespannten Gummiband 40 umfasst, so dass an den beiden Schenkeln 12, 14 eine diese auf einander zu drückende Rückstellkraft anliegt.

Zur einfachen Handhabung des chirurgischen Instrumentes 10 weist der erste Schenkel 12 an seinem dem Stift 20 fernen Ende einen Ring 42 zur Aufnahme eines Zeig- oder Mittelfingers auf. Der zweite Schenkel 14 weist an seinem dem Stift 22 fernen Ende ein Griffteil 44 zur Ausführung von Translationsbewegungen des zweiten Schenkels 14 in Längsrichtung x zur Bedienung mit einem Daumen auf.

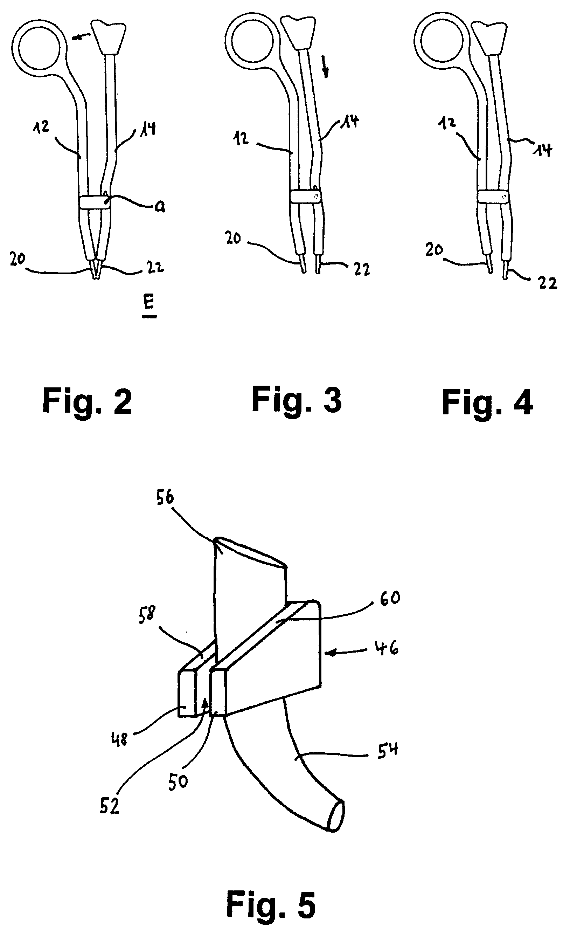

Die Fig. 2 bis 4 zeigen die drei wesentlichen Extremstellungen der Stifte 20, 22 während eines Umstülpvorganges. In Fig. 2 liegen die beiden Stifte 20, 22 unter Bildung einer V-Form mit ihren Spitzen einander an. Diese Nullstellung wird während der Einführung der Stifte in ein umzustülpendes Hohlorgan eingenommen. Fig. 3 zeigt die Spreizstellung der Stifte 20, 22 zur Aufspannung des umzustülpenden Randes des Hohlorgans. In Fig. 4 ist der zweite Schenkel 14 nach einer Translationsbewegung in Längsrichtung x um den Betrag der Länge der Führungsnut 28 gegenüber dem ersten Schenkel 12 verschoben. Durch die entsprechende Bewegung des Stiftes 22 kann ein bereits umgestülpter Teil des Hohlorgans vollständig umgeklappt und richtig platziert werden.

Ein in Fig. 5 gezeigter Amboss 46 weist zwei einander gegenüberliegende Stege 48, 50 mit einer Gesamtbreite von z.B. 1,6 mm auf. Die Stege 48,50 bilden einen Durchführungsschlitz 52 von z.B. 0,8 mm Breite für den endständigen Stumpf 56 eines Hohlorgans 54. Der aus dem Durchführungsschlitz 52 austretende endständige Stumpf 56 wird um die freien Enden 58, 60 der Stege 48, 50 nach aussen umgelegt. Das Hohlorgan 54 ist beispielsweise eine für die Versorgung der linken Brusthälfte mit arteriellem Blut verantwortliche Arterie (Left Internal Mammary Artery, LIMA), die bei einer Koronaranastomose mit einer Koronararterie (Coronary Artery, CA) vernäht wird. Aus strömungstechnischen Gründen werden LIMA und CA in einem Winkel von z.B. 45° vernäht. Diesem Winkel entspricht der Neigungswinkel der freien Enden 58,60 der den Amboss 46 bildenden Stege 48,50. wobei der endständige Stumpf des Hohlorgans entsprechend so zugeschnitten werden muss, dass der umzustülpende Teil des Hohlorgans 54 an den beiden Enden des Ambosses unterschiedlich lang ist. Damit auch der längere Teil des umgestülpten Randes des Hohlorgans dem Amboss wellenfrei anliegt, wird dieser Teil nach dem Umstülpen mit dem Stift 22 durch Ausführen der Translationsbewegung des zweiten Schenkels 14 in Längsrichtung x auf dem Amboss weiter gestossen bzw. glatt gestrichen.

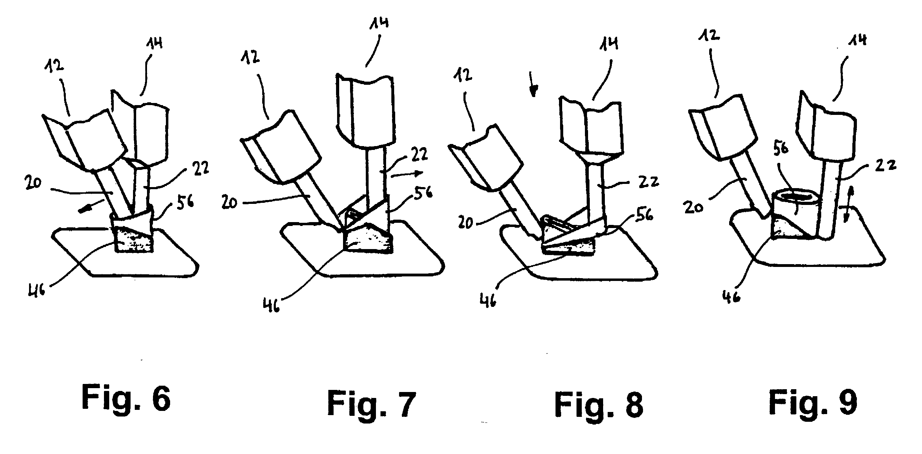

In den Fig. 6 bis 9 ist der Bewegungsablauf der an den freien Enden 16, 18 der Schenkel 12, 14 angeordneten Stifte 20, 22 beim Umstülpen dargestellt.

In Fig. 6 sind die beiden V-förmig aneinander liegenden Stifte 20, 22 in den aus dem Durchführungsschlitz 52 des Ambosses 46 austretenden endständigen Stumpf 56 des Hohlorgans 54 eingeführt. In dieser Stellung wird der Rand des Hohlorgans 54 durch Spreizung der beiden Stifte 20, 22 gedehnt. Wie in Fig. 7 gezeigt, erfolgt zunächst das Umstülpen des kürzeren Teils mittels des am ersten Schenkel 12 angeordneten Stiftes 20. Bei entsprechender Neigung des Stiftes 20 bzw. des Instrumentes 10 gleitet der Rand des Hohlorgans 54 selbsttätig vom Stift. Anschliessend wird gemäss Fig. 8 der Rand des teilweise umgestülpten Hohlorgans 54 durch weiteres Spreizen der Stift 20,22 auf etwa die Länge des Ambosses 46 gedehnt und der länger Teil umgestülpt. Durch zusätzliches Schieben des Stiftes 22 in Längsrichtung x über die oben beschriebene Translationsbewegung des zweiten Schenkels 14 liegt der längere Teil dem Amboss ebenfalls wellenfrei auf (Fig. 9).

## Patentansprüche

1. Chirurgisches Instrument zum Umstülpen eines aus einem von einander gegenüberstehenden und einen Durchführungsschlitz (52) begrenzenden Stegen (48,50) gebildeten Amboss (46) herausragenden endständigen Stumpfes (56) eines Hohlorgans (54) über die freien Enden (58,60) der Stege (48,50) nach aussen,
**gekennzeichnet durch**
zwei von freien Enden (16,18) von zwei eine Längsrichtung (x) definierenden Schenkeln (12,14) abragende und in einer gemeinsamen Ebene (E) liegende Stifte (20,22), wobei ein erster Schenkel (12) über einen in einer im zweiten Schenkel (14) in der Längsrichtung (x) angeordneten Führungsnut (28) gelagerten Führungsbolzen (30) mit senkrecht zur Stiftebene (E) liegender Bolzenachse (a) mit dem zweiten Schenkel (14) so verbunden ist, dass der zweite Schenkel (14) um die Bolzenachse (a) drehbar und relativ zum ersten Schenkel (12) in Längsrichtung (x) verschiebbar ist.

2. Chirurgisches Instrument nach Anspruch 1, **dadurch gekennzeichnet, dass** die beiden Schenkel (12,14) im Bereich zwischen dem Führungsbolzen (30) und den freien Enden (16,18) der Schenkel (12,14) über ein elastisches oder federndes Rückstellelement (40) mit einer die beiden Schenkel (12,14) aufeinander zu drückenden Rückstellkraft beaufschlagt sind.

3. Chirurgisches Instrument nach Anspruch 1 oder 2, **dadurch gekennzeichnet, dass** die beiden Schenkel (12,14) über ein Verbindungselement (24,26) miteinander verbunden sind, wobei das Verbindungselement (24,26) mit dem ersten Schenkel (12) starr und mit dem zweiten Schenkel (14) über den am Verbindungselement (24,26) festgelegten und in die Führungsnut (28) eingreifenden Führungsbolzen (30) verbunden ist.

4. Chirurgisches Instrument nach einem der Ansprüche 1 bis 3, **dadurch gekennzeichnet, dass** die Stifte (20,22) an den freien Enden (16,18) der Schenkel (12,14) auswechselbar angeordnet sind.

5. Chirurgisches Instrument nach einem der Ansprüche 1 bis 4, **dadurch gekennzeichnet, dass** sich die Spitzen der Stifte (20,22) in einer Nullstellung unter Bildung einer V-Form berühren.

6. Chirurgisches Instrument nach einem der Ansprüche 1 bis 5, **dadurch gekennzeichnet, dass** die Stifte (20,22) im Bereich ihrer Spitze auf der von einander weg weisenden Seite eine Ausnehmung (32,34) mit einer im Wesentlichen ebenen Auflagefläche aufweisen.

7. Chirurgisches Instrument nach einem der Ansprüche 1 bis 6, **dadurch gekennzeichnet, dass** der erste Schenkel (12) an seinem dem Stift (20) fernen Ende einen Ring (42) zur Aufnahme eines Zeig- oder Mittelfingers aufweist.

8. Chirurgisches Instrument nach einem der Ansprüche 1 bis 7, **dadurch gekennzeichnet, dass** der zweite Schenkel (14) an seinem dem Stift (22) fernen Ende ein Griffteil (44) zur Ausführung von Translationsbewegungen des zweiten Schenkels (14) in Längsrichtung (x) zur Bedienung mit einem Daumen aufweist.
